# EUROPEAN PATENT APPLICATION

(11) **EP 2 465 358 A1**
(43) Date of publication of application: **20.06.2012**
(21) Application number: 10808053.2
(22) Date of filing: 27.04.2010
(51) Int. Cl.: A23L 1/06, A23L 1/30, A61K 8/04, A61K 8/19, A61K 33/00, A61P 43/00, A61Q 19/00

(54) **METHOD FOR MANUFACTURING A FUNCTIONAL GEL**

(30) Priority: 12.08.2009 JP 2009187409; 14.10.2009 JP 2009237036; 25.02.2010 JP 2010040218
(71) Applicant: Koyama, Kasumi, Tokyo 168-0082 (JP); Murakami, Atsuyoshi, Tokyo 107-0052 (JP); Hanaoka, Kokichi, Ueda-shi Nagano 386-0001 (JP); Ohtsubo, Ryouichi, Tokyo 201-0014 (JP); Matsuo, Yoshiaki, Tokyo 143-0011 (JP)
(72) Inventor: MURAKAMI, Atsuyoshi, Tokyo 107-0052 (JP); HANAOKA, Kokichi, Ueda-shi Nagano 386-0001 (JP); OHTSUBO, Ryouichi, Komae-shi Tokyo 201-0014 (JP); MATSUO, Yoshiaki, Tokyo 143-0011 (JP)
(74) Representative: Marconnet, Sébastien
(86) International application number: PCT/JP2010/003019
(87) International publication number: WO 2011/018865

(57) **Abstract**

At first fill the airtight container 2 with water (solution), and supply a gas such as hydrogen from the gas supply pipe 5, drive the supersonic wave vibratory plate 4, then the micro-bubbles 1b of the gas are generated. The diameter of the micro-bubbles 1b is for example nanosize, then the speed of rise in the water is slow, so it remains underwater for about 10 minutes even if the supersonic wave vibratory plate 6 is stopped.

## Description

### TECHNICAL FIELD

This invention relates to a production method of a functional gel containing minute air bubbles inside.

### BACKGROUND ART

It is thought that hydrogen has a function to neutralize active oxygen causing aging. Therefore, a hydrogen water which encloses a bubble of the hydrogen gas in water and a gel into which are kneaded bubbles of the hydrogen gas in solution of high viscosity are proposed in Prior art 1-4 conventionally.
A manufacturing method of an oleaginous gel (W/O type emulsion) and a manufacturing method of an aqueous gel (O/W type emulsion) are described in Prior art 1, as the manufacturing method of the oleaginous gel, a process to make water in which extra hydrogen gas dissolves and a process to dissolve a water-soluble macromolecule (a gelling agent) in the water are described.

And a gel which kneads / blends bubbles of hydrogen gas into a solution of high viscosity is suggested in Prior art 2-4. Specifically, Prior art 2 describes a production method of a liquid into which gas is dissolved putting gas such as hydrogen gas in a raw materials liquid through a static mixer, and a gelatinous drink is exemplified as one of the raw material liquids.

Prior art 3 describes a hydrogen colloid of the viscous solution. It produces micro-bubble of hydrogen gas continually using a supersonic wave vibration system, sends this micro-bubble of hydrogen gas to homogenization equipment, kneads the micro-bubble of the hydrogen gas into a viscous solution, and the hydrogen colloid is manufactured.

Prior art 4 describes a manufacturing method of gelatinous food holding hydrogen gas inside. In this method, raw materials of the jelly are put in a screw type stirrer and hydrogen gas is supplied to the jelly from the stirrer, finally this is given a sugarcoating.

### PRIOR ART

### PATENTED DOCUMENTS

[Patent Document 1] Japanese laid open 2007-3 14496 bulletin
[Patent Document 2] Utility model registration No.3139460 bulletin
[Patent Document 3] Japanese laid open 2008-279424 bulletin
[Patent Document 4] Utility model registration No.3106002 bulletin

### DISCLOSURE OF THE INVENTION

### OBJECT OF THE INVENTION

Prior art 1 does not intend to leave long term uniform micro-bubbles in a gel, because it describes the removal of the gas which was mixed in the manufacturing process from the gel.
And in Prior art 1, it is intended to manufacture a gel which hydrogen gas or atomic hydrogen dissolves. Therefore, in the first process, making hydrogen gas with a micro-bubble of 2µm - 120µm, it assumes a quantity of dissolved hydrogen of the hydrogen added water to be 0.5ppm - 1.5ppm. In the second process after the first process, it dissolves a water-soluble macromolecule, but a lot of the hydrogen gas is lost into the air at the stage of changing from the first process to the second process, then most of the bubbles of the hydrogen gas do not remain in the final product.

Also, in the subject matter described in Prior art 2-4, that is the blending of a gas into a solution of high viscosity, control of the bubbles becomes difficult as they grow both larger and fragmented.

In Prior art 2, it is extremely difficult to scatter micro-bubbles of hydrogen uniformly because hydrogen gas is fed into a highly viscous gel.
In Prior art 3, bubbles do not escape over a short time because bubbles of the hydrogen gas are kneaded into a solution of high viscosity but, when making micro-bubbles using a supersonic wave vibration system, they cannot be spread uniformly. In Prior art 3, it is described that a homogeneous hydrogen colloid is provided. However, as a practical matter, it is difficult to distribute these uniformly.
Also as for Prior art 4, likely to Prior art 2 and 3, It is extremely difficult to spread micro-bubbles uniformly, because feeds micro-bubbles of hydrogen are fed into highly viscous materials at a late stage.

In addition, none of the prior arts solve the real problems of mass-production, such as how to gelate consecutively or how to avoid contact with the air.

### MEANS FOR SOLVING THE PROBLEM

In order to solve the above mentioned problems, a manufacturing method of a functional gel corresponding to the first invention of the present application is outlined below:
Put water or a low viscosity solution into a container, feed hydrogen gas into the water or the low viscosity solution and generate minute hydrogen gas bubbles, feed the water or the low viscosity solution in which the minute hydrogen gas bubbles are generated into an adjacent container using a tubular pump, add a gelling agent into the adjacent container while the micro-bubbles remain in the water or the water solution, gelate the water or the solution and hold / retain the micro-bubbles of the hydrogen gas inside.

And a manufacturing method of a functional gel corresponding to the second invention of the present application is outlined below:
Put water or a low viscosity solution into a container, feed a gas (except hydrogen gas) into the water or the low viscosity solution and generate minute gas bubbles, feed the water or the low viscosity solution in which minute gas bubbles are generated into an adjacent container using a tubular pump, add a gelling agent into the adjacent container while the micro-bubbles remain in the water or the solution, gelate the water or the solution and hold micro-bubbles of the hydrogen gas inside.

And a manufacturing method of a functional gel corresponding to the third invention of the present application is outlined below:
Put water or a low viscosity solution into a container, feed a hydrogen gas into the water or the low viscosity solution and generate minute gas bubbles, feed the water or the low viscosity solution in which minute gas bubbles are generated into an adjacent container using a tubular pump, a gelling agent is put in the adjacent container beforehand, mix the gelling agent in the adjacent container and the water or the low viscosity solution including minute hydrogen bubbles, gelate the water or the solution and hold the micro-bubbles of the hydrogen gas inside.

In the above manufacturing method of a functional gel, if an enteric coated material which is hard to dissolve in acid and easy to dissolve in alkali is dissolved in the water solution, when it is used in functional foods, hydrogen will arrive at the small intestine without being broken down, it is thought that the small intestine takes in hydrogen.

In the present invention, it is expected that vitamins, various nutritional supplements, including ineral in water solutions, and the low viscosity solution means not only a water solution but also one with an organic solvent (alcohol, ether, benzene, acetone, toluene, hexane), or low viscosity oils and fats (saturated fatty acid, unsaturated fatty acid) solvent.

### EFFECT OF THE INVENTION

When using or supplying a gas in a liquid state it is very difficult to control the gas sealed in a tank in a high pressure state, However, the present invention makes it possible.
According to the manufacturing method of the functional gel of the present invention, because it adds a gelling agent in the same process as generating minute hydrogen gas bubbles, It can gelate a water (solution) before the generated hydrogen gas bubbles escape from the water (solution), also it can produce contained micro-bubbles, such as hydrogen, in a gel uniformly besides controlling the quantity of the micro-bubbles.
Also, once the product has been made, because the internal micro-bubbles remain in the gel, it is convenient to carry and easy to sell.
In addition, when used for functional foods, a texture is preferable, and when used for cosmetics, it is easy to apply to the skin.

Gels which contain bubbles inside have the following functions:
(1) Gas is hard to separate from the gel unless the gel melts. The gas is not released into the atmosphere in the range where the gel does not melt, that is at normal temperatures. Therefore, the gas can be carried or handled like a solid under normal temperature conditions.
(2) It is necessary to maintain a cryogenic temperature state to hold gas in a liquid state. It is possible to hold the gas under normal temperature conditions unless they reach a condition where the gel melts.
(3) When the gas contained in gel has virulence and ignition characteristics, it is necessary to take measures that prepare for an emergency, but storage in a tank or other facilities is easier when in gel form than if either in a liquid or gas state.
(4) In the case of a virulent gas, held in a liquid or gas state, were it to leak it could disperse widely in the atmosphere, and the management, transportation and general work environment endangered. However, when the gas is held in the gel, dispersion to the atmosphere becomes more difficult.
(5) This is symbolized by the difficulty in controlling the engine of a space rocket which mixes liquid oxygen and liquid hydrogen. When it uses gas at room temperature it is very difficult to control the timing of the vaporization from a liquid that is to be released from a tank. Also, it is difficult to directly control the gas in a liquid state.
   However, if the gas is contained in a gel, it can be maintained at room temperature in the gel with adjusting gaseous density and mixture ratio, thus handling becomes much easier.
(6) When using gases directly after mixing, controlling the rate of increase of the gases becomes difficult. In particular, it is almost impossible to use a mixed gas in the form of either a liquid or a gas in the living bodies of animals and plants. When intending to use gas in small quantities in vivo over a long time it is difficult to do so with a tank and/or an apparatus. Besides, using a plurality of gases in a small quantity over a long time is full of difficulty.
   In contrast, a gas can be supplied for a long stretch of time, in small quantities, in vivo by using a gel containing gaseous bubbles, such as by combining a plurality of gels each containing a single gas.
   Alternatively, by forming the gel into the shape of a cylindrical layer, like a Baumkuchen or Swiss Roll, the gas can be completely generated within an elapsed time frame.
(7) By putting the gel containing bubbles in a capsule (for example, enteric coated capsule), the gel containing bubbles can be delivered to a particular place. Also, by coating the objective part or inserting the gel into the objective part directly and dissolving the gel therein, gas can be supplied to the objective part.

The present invention shows the following effects on preparing the gel having the above functions (1) - (7).
Firstly, bubbles which are small and uniformly dispersed can be obtained, since the bubbles are generated in water (solution) before gelating, and bubble content per unit volume of the gel can be adjusted.
Also, as the airtight container and the gelating container are separated, the functional gel is manufactured consecutively, for example, by setting a lot of gelating containers on a turntable or conveyer belt, once the water (solution) containing the bubbles has been filled into one gelating container, the pipe, which feeds the water (solution) containing bubbles or high pressure water, is changed to the following gelating container and so on.
In addition, since the water (solution) is not exposed to the air, oxidation, a change in quality or contamination can be prevented. It is highly suitable as a manufacturing process of supplements.

### BRIEF DESCRIPTION OF DRAWINGS

[Fig.1] A figure showing a condition of a function gel obtained by the present invention spilling out of /being ejected from a container.
[Fig. 2] An enlarged view of a functional gel provided by the present invention method.
[Fig. 3] (a)∼(c) is a figure showing the manufacturing process of the functional gel.
[Fig. 4] A figure explaining a manufacturing method of the functional gel of the present invention.
[Fig. 5] A figure explaining a manufacturing method of the functional gel of the other embodiment.

### PREFERRED EMBODIMENT OF THE INVENTION

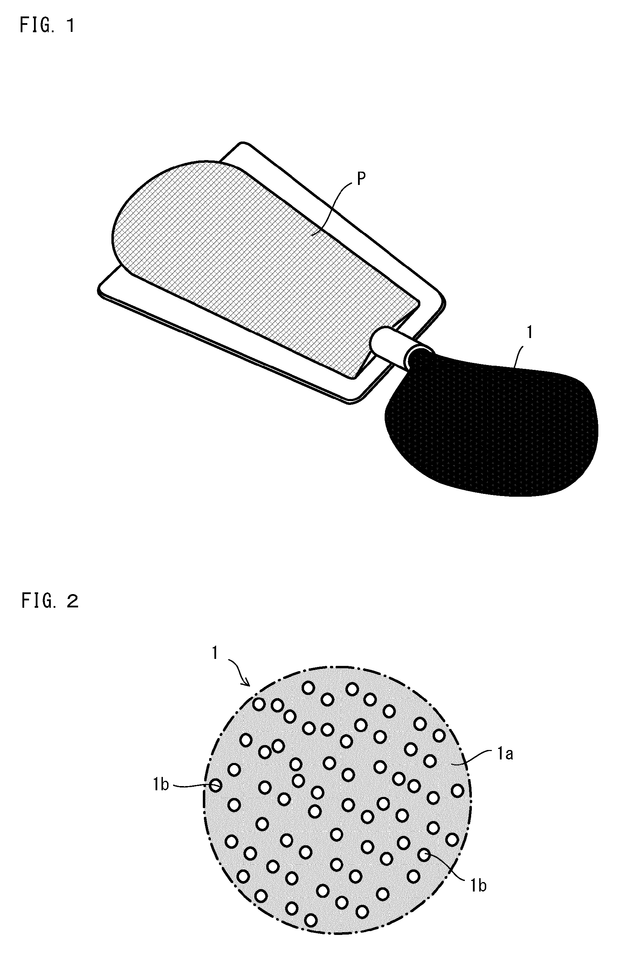

As shown in FIG 1, functional gel 1 provided by the present invention method, because the shape is highly flexible, it can put in a case P made from plastic or aluminum. Alternatively, the shape of the case is arbitrary as well as shown in Fig1.

As shown in FIG 2, as for functional gel 1, micro-bubble 1b of hydrogen disperses uniformly in gel 1a as the continuous phase.

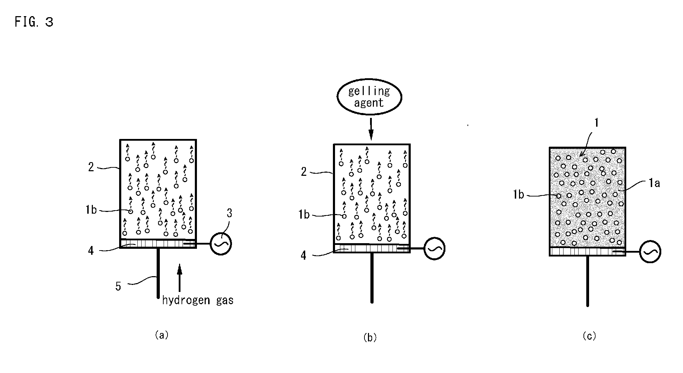

Fig 3 (a) - (c) is a figure showing the manufacturing process of a functional gel, (a) - (c) is a sequence process performed in one container. A supersonic wave vibratory plate 4 connected to high frequency source 3 is arranged in the bottom of container 2, a gas supply pipe 5, for a gas such as hydrogen, penetrates through the supersonic wave vibratory plate 4 and opens in container 2 from the lower part.

As for means to generate micro-bubbles, as well as a supersonic wave vibratory plate 4, even a porous plate, a stirrer, a jet spray nozzle or an aspirator are possible.

For manufacturing the functional gel, as shown in Fig (a), first fill the airtight container 2 with water (solution), supply a gas such as hydrogen from the gas supply pipe 5, and drive the supersonic wave vibratory plate 4, then the micro-bubbles 1b of the gas are generated.
If the diameter of the micro-bubbles 1b is for example nanosize, then the speed of rise in the water is slow, so it remains underwater for about 10 minutes even if the supersonic wave vibratory plate 6 is stopped.

Then as shown in Fig 3 (b), add a gelling agent while the micro-bubbles 1b remain underwater. In this case, what may stop even if continue the generation of micro-bubbles 1b of gas such as hydrogen.

A gelling agent may include the following,
Almond gum, gum elemi resin, danmaru resin, arabian gum, karaya gum, thoragantt gum, arabini galacta, gady gum, momo resin, ama seed gum, guar gum enzyme resolvent, tamarind seed gum, kasia gum cyryume seed gum, thalla gum, karo bean gum (locust bean gum), desert mugwort seed gum, riacansos gum, a guar gum, sesbania aculeata gum, alginic acid, a bag paste extract, furseleran, carrageenan, aloe seawife extract, kidachi aloe extract, pectin, okra extract, hibiscus, aeromonas gum, enterobacter gum, bacillus natto gum, aureobasisium culture fluid, curdlan, pullulan, azotobacter vinerangy gum, xanthan gum, macrohomosexual psis gum, weran gum, jeran gum, ramzan gum, elwinia gum, mitsuencis gum, sklero gum, levan, enterobacter sima eggplant gum, dexistran, yeast cell membrane, chitin, oligo glucosamine, form of microfilament cellulose, chitosan, crystallite cellulose, glucosamine, agar, soy bean polysaccharides, nata de coco, starch, konnyaku potato extract, gelatine, lectin.
Also, vitamins or a mineral may be included besides a gelling agent to improve functionality.

Also, the gelling agent except the above includes the following.
(1) An oil gelling agent of the low molecular compound
   1,3:2,4-dibenzylidene-D-sorbitol, 12-hydroxy stearic acid, N-rawloil-L-glutaminic acid, - α, γ - bis-n-butyramide, spin labeled steroid, cholesterol derivative, dialkyl phosphoric acid aluminum, phenolic cyclic oligomer, 3-bis-n-hexadeciLoki cyan thorasen, cyclic dibusi peptide, partial fluorination alkane, cystine derivative, bis(2-ethyl hexyl) sulfosuccinic acid sodium, triphenyl amine derivative, butyrolactone derivative, the fourth grade ammonium salt, fluorination alkylation oligomer, urea derivative, vitamin H derivative, gulcon amid derivative, cholic acid derivative.
(2) An amino acid-based oil gelling agent
   L-isoleucine derivative, L-valine derivative
(3) A cyclic dipeptide type oil gelling agent
   2,5-di keto piperazine derivative, cyclic dipeptide (composed of a neutral amino acid (L-valine, L-leucine, L-phenylalanine) and an acid amino acid (L-glutaminic acid-γ-ester, L-aspartic acid-β-ester)
(4)An oil gelling agent of the cyclohexane diamine derivative
   diamide (complex from 1,2- trans type cycrohexanediamine), urea derivative (complex from 1,2- trans type cycrohexanediamine).
(5) An oil gelling agent of the dual sovereignty type amino acid derivative dual sovereignty type L- isoleucine derivative, dual sovereignty type L-valine derivative.
(6) Other oil gelling agents
   1,3;2,4- dibenzylidene-D-sorbitol,
   N-rauloilL-glutaminic acid-α,γ-bis-n-butyramide, benzoil gulcon amid derivative, L-isoleucine derivative,
   L-valyl -L- valine derivative, diamide (complex from 1,2- trans type cycrohexanediamine), di urea derivative (complex from 1,2- trans type cycrohexanediamine), dual sovereignty type amino acid derivative, L-lysine derivative, O-methyl-4, 6-benzylidene-D-galactose, 2,3-O-isopropylidene glyceraldehyde derivative.
(7) A hydro gelling agent of the amino acid derivative dibenzoyl-L-cystine, L-cystine derivative, compound (dissolved only in phosphate buffer solution) which bridged glutaminic acid monoester in diisosianate, dual sovereignty type amino acid derivative which bridged L-leucine, L-valine, L-phenylglycine with oxalic acid, organic gelling agent (it covers with a positive charge or a negative charge) which assumed L-lysine a base, hydrogelling agent which assumed an ethanol compound having a pyridine group, L-serine a base,
(8) A hydro gelling agent including the sugar
   D-lactose derivative, D-maltose derivative, D-glucose derivative,
   D-mannose derivative, D-galactose derivative, azobenzene compound.
(9) Other hydro gelling agents
   aporol typed dendrimer, nucleotide containing dual sovereignty type hydrogelling agent, bile acid derivative, vancomycin, dendrimer which has phosphorus in the center, rexren derivative, hydrogel comprising dual sovereignty type surfactants, cationic glutaminic acid compound having naphthalene sulfonic acid, deoxyuridine compound.
(10) A temperature-responsive macromolecule
   polyethylene glycol / polypropylene glycol copolymer,
   polyethers (polyethylene oxide = PEO, poly (EO/PO) copolymer), PEO-PPO-PEO triblock surfactant, alkil PEO surfactant, poly(vinyl methyl ether) = PVME, poly(oxyethylene vinyl ether) = POE VE), hydroxyl propyllyerate, cellulose derivative (hydroxypropyl methylcellulose, hydroxypropyl cellulose, methylcellulose), polyvinyl alcohol derivative, poly(N-substituted acrylic amide) derivative (poly(N-substitution acrylic amide), poly(N-vinyl pyrrolidone), a poly (ethylokusazorin), poly(N-vinyl isobutyl amide) = PNVIBA, poly(2-carboxy isopropyl acrylic amide) = PCIPAAm, poly(N-isopropyl amide).

An enteric coated material includes the following, methacrylate copolymer, hydroxyl propyl methyl cellulose phthalate, hydroxyl propyl methyl cellulose acetate succinate, carboxy methyl cellulose (CMEC), cellulose acetate phthalate, cellulose acetate trimellitate, methacrylate - acrylic acid ethyl ester copolymer, methacrylate - methyl methacrylate ester copolymer, propylene glycol, sorbitan monolaurate, acetic acid phthalate cellulose (CAP), acetic acid trimelito acid cellulose, phthalate hydroxypropyl methylcellulose (HPMCP), methacrylate, chitosan, mannogalactan, pectin, low refuse bean gum, polyethylene glycol (PEG), shellac.

As shown in FIG 3 (c), when a gelling agent is added, water (solution) becomes a gel 1a, and micro-bubbles 1b are uniformly locked inside.

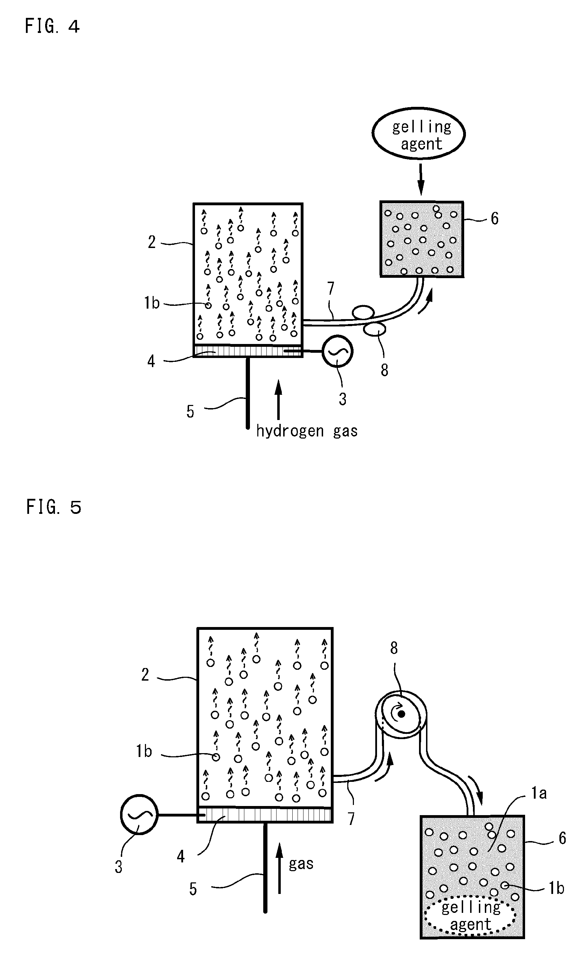

Fig 4 shows a manufacturing method of the functional gel of the present invention, in this embodiment, provide a container 6 for gelation adjacent to the container 2 which generates micro-bubbles of gas such as hydrogen in water (solution), sustain the container 2 and the adjacent container 6 for gelation by a flexible pipe 7, feed the water (solution) including the micro-bubbles in the container 2 into the container 6, and add a gelling agent o the water (solution) in the container 6 using tubular pump 8. As for the tubular pump 8, for example WELCO Co., Ltd. type WP1000 can be applied.

According the above-mentioned process, micro-bubbles dispersed uniformly are provided in the gel 10 as the continuous phase.

Also,the following adjustment can be performed in the middle of the gelation or after the gelation.
(1) addition of the gelation regulator
(2) heating
(3) cooling
(4) irradiation of light, radiation, electromagnetic wave including a radio wave
(5) irradiation of sound wave, supersonic wave
(6) oscillation and application of physical pressure
(7) bleaching in an electric field, or a magnetic field
(8) application of a voltage
(9) adjustment of pH

Herein, there are limitations as to the type of gas supplied from the gas supply pipe 5:
The following (a) - (e) are examples and it is possible to mix the examples.
   (a) Flammable gas, Toxic gas,
   (b) Firedamp,
   (c) Non-inflammable gas,
   (d) Water-soluble gas,
   (e) Non-water-soluble gas

Gas classified in (a) includes the following;
Methane, Propane, n butane, i butane, n pentane, Ethylene, Propylene, Butylene, Acetylene, Toluene, O xylene, Methanol, Ethanol, Acetone, Methyl ethyl ketone, Ethyl acetate, Butyl acetate, City gas (a methane basis), LPG( isobutane basis), Gasoline, Kerosene, n xanthan, Butadiene, Acetaldehyde, Chloroethylene, Carbon monoxide, Ammonia, Hydrogen sulfide, Chlorine, Sulphur dioxide, Benzene, Acrylonitrile, Methyl bromide, Ethylene oxide, Hydrogen cyanide, Carbon oxychloride, A hydrogen chloride, Arsine, Phosphine, Silane, Diborane, German, Diblack Lucien, Selenium hydrogen, Fluorine, nitrogen dioxide, 3 fluorinated chlorine, Hydrogen fluoride, hydrogen bromide, Ozone.

Gas classified (Ignition degree G1 - G5, explosion class 1-3) in (b) includes the following; Acetone,
Ammonia, Carbon monoxide, Ethane, Acetic acid, Ethyl acetate, Toluene, Propane, Benzene, Methanol, Methane, Coal gas, Water gas, Hydrogen, Ethanol, Acetic acid isopentyl, 1 butanol, Butane, Acetic anhydride, Ethylene, Ethylene oxide, Acetylene, Gasoline, Hexane, Acetaldehyde, Ethyl ether, Carbon disulfide.
The classification by the international regulations of the International Electric standard meeting (IEC), a temperature class is T1 - T5, group IIA - IIC, following;
Acetone, Ammonia, Carbon monoxide, Ethyl acetate, Toluene, Propane, Benzene, Methanol, Methane, LP gas, Ethane, Acetic acid, City gas, Hydrogen, Ethanol, i butane, 1 butanol, Acetic acid isobentill, Acetic anhydride, Ethylene, Ethylene oxide, Acetylene, Gasoline, n hexane, Acetaldehyde, Ethyl ether, Carbon disulfide.

The gas classified in (c) includes all "toxic gas", "flammable gas" other than "special materials gas" determined by the law.
The special materials gas is used in semiconductor production, a reactivity of this gas is thought to be highly dangerous and comprises the following 39 kinds.
A silane group: Monosilane (SiH4), Disilane (Si2H6), Dichlor silane (SiH2Cl2), Trichloride silane (SiHCl3), Silicon tetrachloride (SiCl4), Silicon tetrafluoride (SiF4).
An arsenic group; Arsine (AsH3), 3 fluorinated arsenic (AsF3), 5 fluorinated arsenic (AsF5), Arsenic trichloride (AsCl3), 5 chlorination arsenic (AsCl5). A phosphorus group; Phosphine (PH3), 3 fluorinated phosphorus (PF3), 5 fluorinated phosphorus (PF5), Trichloride phosphorus (PCl3), 5 phosphorus chloride (PCl5), Oxy phosphorus chloride (POCl3).
A boron group; Diborane (B2H6), 3 fluorinated boron (BF3), Trichloride boron (BC13), 3 boron (BBr3) bromide.
Metal hydride; Selenium hydrogen (H2Se), Mono German (GeH4), Tellurium hydrogen (H2Te), Stibine (SbH3), Hydrogenation Tin (SnH4). Halide; 3 fluorinated nitrogen (NF3), 4 fluorinated sulfur (SF4), Tungsten hexafluoride (WF6), 6 fluorinated molybdenum (MoF6), Tetrachloride germanium (GeCl4), Tin tetrachloride (SnCl4), 5 chlorination antimony (SbCl5), 6 chlorination tungsten (WCl6), 5 chlorination molybdenum (MoCl5).
Metal alkylate; Trialkyl gallium (Ga(CH3)3, Ga(C2H5)3), Trialkyl indium (In(CH3)3, In(C2H5)3).
And 7 kinds of gas those are Monosilane (SiH4), Disilane (Si2H6), Arsine (AsH3), Phosphine (PH3), Diborane (B2H6), Selenium hydrogen (H2Se) and Mono German (GeH4), these are prohibited to carry-on / transport by air in a high pressure state (more than IMPa [10 standard atmosphere]), and are referred to as "special high-pressure gas".
Arsenic group, Phosphine (PH3), 3 fluorinated phosphorus (PF3), Trichloride phosphorus (PCl3), 5 phosphorus chloride (PCl5), Oxyphosphorus chloride (POCl3), Diborane (B2H6), 3 fluorinated boron (BF3), Trichloride boron (BC13), Selenium hydrogen (H2Se), 4 fluorinated sulfur (SF4) are specified to poison / drastic medicine.
A toxic gas is a harmful gas, and the permitted density refers to less than 200ppm, the list is shown below.
Acrylonitrile (C2H3CN), Sulfurous acid gas (S02), Ammonia (NH3), Carbon monoxide (CO), Hydrogen chloride (HCl), Chlorine (Cl2), Chlor methyl (CH3Cl), Hydrogen cyanide (HCN), Carbon disulfide (CS2), Fluorine (F2), Blom methyl (CH3Br), Benzene (C6H6), Carbon oxychloride (COCl2), Hydrogen sulfide (H2S).
Hydrogen cyanide (HCN) is specified as being a poison. Acrylonitrile (C2H3CN), Ammonia (NH3), Hydrogen chloride (HCl), chlorine (C12), Carbon disulfide (CS2), Blom methyl (CH3Br) are specified as being drastic medicines.
A flammable gas is defined as one which when it mixes with air the lower limit of the explosion limit is less than 10% and the difference between the upper and lower limit of the explosion limit more than 20%, and It includes the following.
Acrylonitrile (C2H3CN), Acetylene (C2H2), Amonia (NH3), Carbon monoxide (CO), Ethylene (C2H4), Chlor methyl (CH3Cl), Hydrogen cyanide (HCN), Cyclopropane (C3H6), Hydrogen (H2), Carbon disulfide (CS2), Butadiene (C4H6), Butane (C4H10), Propane (C3H8), Propylene (C3H6), Blom methyl (CH3Br), Benzene (C6H6), Methane (CH4), Methyl ether ((CH3)2O), Hydrogen sulfide (H2S).
Ammonia (NH3), carbon disulfide (CS2) and Blom methyl (CH3Br) are specified as being drastic medicines.

Gases classified in (d) includes the following commonly used gases: Oxygen, Nitrogen, Argon, Methane, Hydrogen, Acetylene, Hydrogen chloride, Carbon dioxide, Nitrous oxide, Various high-pressure gas (hydrogen sulfide, LP gas, propane, ammonia, chlorine, sulfurous acid gas, butane, hydrogen cyanide, ethylene oxide).

Gas classified in (e) includes bromine displayed other than the above described gas group in the periodic table.

A usage of the hydrogen gel is described above and a specific usage of an oxygen gel, an ozone gel, a nitrogen oxide gel, a carbon dioxide gel, a helium gel, a chlorine gel, a bromine gel are describes below.

### [Oxygen gel]

As the gaseous quantity dissolved in water is extremely small, then it is not available in large quantities for normal usage like gas stored in a tank under high-pressure conditions.
It is available if the gas is liquefied under a special environment, but, there is not such a thing in the normal environment.
Therein, by making nano or micro bubbles of a gas in water, and adding a gelling agent while the bubbles rise slowly in the water for about ten minutes, a large quantity of gas can be fixed in the gel.
Also, the oxygen density in the air bubble can be regulated freely. In addition, mixed gases may be used by adding other gas to the oxygen. When oxygen is selected as a gas, oxygen of the nano or micro sized bubbles exist in the gel in large quantities.
When the materials of the gel are made water-soluble, oxygen can be generated by adding water.
Also, in adding insolubility and insoluble material to the gel, the gel gradually dissolves to water and will gradually supply oxygen. Herein after referring to this gel as (OTRG = Oxygen Time Releasing Gel).

### OTRG Example 1

For a water tank in which to carry a fish it is necessary to continue supplying oxygen to the fish by using motor pumps.
However, when the oxygen supply is stopped by electrical or mechanical trouble, the fish in the water tank suffocates and it is difficult to save it. Then, by providing OTRG to the water in the tank oxygen can continue to be supplied.
By accident, when spend OTRG of the glut, under 1 standard atmosphere, more than oxygen of the fixed quantity do not dissolve escaping outside a container, it does not become the hyper oxygen state. If, by accident, too much OTRG is added to the water in the tank, under 1 standard atmosphere, it will create a hyper oxygen situation as any surplus oxygen dissolved will escape from the container.

### OTRG Example 2

For a person who suffers from a lowering in their blood oxygen saturation level through disease, it is necessary to raise the saturation level by supplying oxygen of a density (20 → 30%) that is higher than existing oxygen in normal gas in the body using an oxygen mask or oxygen supply. However, conventional apparatus supplying oxygen requires an oxygen cylinder or transmission membrane, there is a disadvantage in that this type of apparatus is bulky.
Therein as one example, a small and simple mask using OTRG to supply the oxygen is envisaged.
This mask becomes the perfect oxygen feed means for a person who going to go mountain climbing at high altitudes, also as an emergency oxygen mask installed in an airoplane.

### OTRG Example 3

OTRG can be use as a supplement.
Currently, water which is said to include a lot more dissolved oxygen than normal water, it is called "Oxygen Water", is manufactured and sold all over the world,
Depending on temperature, normal water includes dissolved oxygen of 3 - 5mg/ liter, however according to the makers of "Oxygen Water", they say that "Oxygen Water" has an oxygen content which is 10 ∼ 40 times that of ordinary water.
Oxygen is supplied to the body by taking in water including oxygen in large quantities, then the makers of Oxygen Water say that following effects are expected.
(1) Activation of brain cells, improved concentration and dispels
   drowsiness.
(2) Immunostimulation
(3) Promotion of fatigue recovery by resolving fatigue material "lactic acid" generated by exercise.
(4) A diet effect can be expected because it promotes lipolysis.
(5) As seen in oxygen therapy, performs lively metabolism of the skin by stimulating skin cells.
However, after drinking "Oxygen Water", a fall in the oxygen content of water will happen with heating up in the body. Thus, most oxygen vaporizes in the stomach, therefore, the quantity of oxygen to enter the bowels or blood is extremely small.
As a result the effect of "Oxygen water" is difficult to measure and there are a great many medical experts who question the effect.
On the other hand, OTRG is dissolved inside of the stomach when it it is ingested and oxygen is generated in the stomach.
However, when enteric coated gel is assumed or the gel is put in an enteric coated capsule, OTRG does not generate oxygen in the stomach but generates oxygen in the bowels, it can easily perform the supplying of oxygen only to the bowels..
Therefore, in comparison with "Oxygen Water", increased oxygen is precisely supplied to the body.
Also, with OTRG which includes nano or micro sized oxygen bubbles, it is extremely easy to set the oxygenicity per unit capacity as several times or more that of "Oxygen water".
And, OTRG can continue to generate oxygen in the bowels for a long time. It is known that by taking in 500mL of air in one breath approximately 100mL of oxygen is taken into the body, therefore, many articles deny that the taking of Oxygen Water has the capacity to improve athletic performance.
However, for competitions such as track, swimming, particularly in short distance competition in which apnea / shallow breathing is performed, the gel can be expected to play a role the supplying of internal oxygen.
It is impossible to drink a large quantity of oxygen containing water just before the start of a race, but, it is easy for the body to supply oxygen by combining an oxygen gel and an enteric coated capsule.

### OTRG Example 4

The gel of the present invention can be coated on the skin or surface of animals or plants. It can also be inserted into their internal organs /structure. The gel of the present invention can be applied to those parts of the body where anaerobic bacteria, a mold or a fungus propagate, such as the palate which is not exposed to atmospheric oxygen, and to wounds such as open wounds, abrasions and contusions, for example cuts, puncture or gunshot wounds, bites, grazes and bruises.
It is possible to kill or weaken an anaerobic microbe by coating or putting the OTRG on the part which is not exposed to the atmosphere, or to insert the OTRG in the form of a capsule or tablet.
Also, pharmaceutical products including an antibiotic, medicinal properties of an unregulated drug, cosmetics, and ingredients from material extracted from animals or plants may be mixed with the gel.
According to the medical data, it is known that a wound heals more quickly if exposed to atmospheric oxygen rather than being covered after stitching. It is believed that capillary reproduction is hastened by exposure to oxygen. Therefore, a hyperbaric oxygen therapy is utilized.
Hyperbaric oxygen therapy is effective in the treatment of wounds, however, the facilities required, such as tanks, are large-scale and downsizing is difficult,
In addition, the use of high-pressure oxygen is difficult to manage.
In normal circumstances, it is not portable and is inconvenient.
Therein, by coating or injecting /spreading OTRG on the part of the wound that does not come into contact with the atmosphere, the promotion of capillary reproduction can be expected. As, at this time, the oxygen density of the bubbles contained in the gel can be adjusted. Mixed gases which combined oxygen and other gases can be used..
In addition, an ingredient for connective tissue reproduction such as hyaluronic acid, collagen, elastin or placenta extract may be used in combination as an ingredient of the gel.

### OTRG Example 5

OTRG is valid as a combustion accelerant by combining an oxygen gel with inflammables. Also, OTRG is available as an oxidation promotion material.

### OTRG Example 6

In the case of scuba diving, a human being cannot stay underwater for any length of time without using an oxygen cylinder.
Usually, a maximum of about 200 standard atmosphere air is compressed in the cylinder depending on the depth of the descent. Normally, the oxygen in the cylinder will be used up in 1 ∼ 1.5 hours and the person using the cylinder must return to the surface.
Therein, by dissolving the oxygen gel of the present invention in the cylinder or an adjacent place by heating, adding water or making a chemical change, extracting the oxygen held in the bubbles and adding this oxygen to the air in the tank, the time able to be spent under the water can be increased dramatically.

### OTRG Example 7

OTRG can be used as a fuel for a fuel cell in combination with hydrogen gel. In this case, dissolve hydrogen and oxygen in water under an environment of several standard atmospheres or dozens of standard atmospheres, and generate micron or nano sized bubbles, then a water gel of high viscosity containing an increased quantity of hydrogen and oxygen can be produced.

### [Ozone gel]

A gel which contains micro-bubbles of ozone may be effective in inactivating /countering a norovirus, that is, an ozone gel dissolves and ozone gas is released, then this ozone gas is resolved to oxygen, a free radical is generated, and this free radical has disinfectant properties. In this case, the bubbles of ozone gas have a negative electric charge even if the density of the ozone gas is low, on the other hand, many viruses are positively charged, therefore, the virus is drawn to the bubbles of the ozone gas and sterilized.

### [Nitrogen gel]

Nitric oxide (NO) is attracting attention recently. Because of its vasodilation properties it may be available for pharmaceutical products for cardiovascular system diseases and ED medical treatment.
Also, bodybuilders use NO in their attempt to build muscle too.
(1) In taking a controlled release related gel or capsule which includes large quantities of nano or micro sized nitric oxide bubbles, muscle increase / building, ED cancellation is assisted..
(2) A macrophage around a cancer cell produces NO, this NO paralyzes the mitochondrial function of the cancer cell and kills the cancer cell by disturbing the DNA composition.
(3) There is a disadvantage to reducing NO when administering insulin in the medical treatment of diabetes.
(4) NO gas has a role in the transmission of signals in the brain.
(5) In the case of using a nitrogen gel for container and building materials, the gel is dissolved when the temperature rises, and it can keep fire prevention characteristics.
(6) A nitrogen gel can be use as an antioxidant.
Therein, give its controlled release characteristics, by combining a nitrogen gel and an enteric coated capsule it is useful in fields such as sports and / or medicine as in the above described (1) - (4).
Also, by coating or injecting the gel to the necessary parts directly (e.g., the outer tissue of a cancer and an animal organ or plant cell) it is expected to achieve a medical effect on the above described (1) ∼ (4) and (6)

### [Carbon dioxide gel]

(1) Use as a cosmetic to attempt blood circulation promotion and activation of the skin.
(2) By putting the gel in drinking water it can easily make a carbonated drink. It can also restore the flavor of a carbonated drink and a low-malt beer by replacing the carbonic acid which has been lost.
(3) A large quantity of carbon dioxide gel functions as a fire prevention / retardant at a time of high heat generation and as an extinguishant at a time of fire generation. For example, a seat cover which is coated with carbon dioxide gel would be efficient in putting out a fire since the cover, if put over the source at the time that the fire started in a kitchen, would not only intercept oxygen but would also generate carbon dioxide.
(4) The carbon dioxide is known as "breathing excitence", it is used for the medical treatment of the hyperventilation syndrome, therefore, the carbon dioxide gel is available effectively for a person needing breathing excitancy other than a hyperventilation syndrome.
(5) In the case of the oxygen absorption with the oxygen cylinder which is used for scuba diving and medical care, it is known that when mixes 5-10% of carbon dioxide is more effective than oxygen alone, therefore, it can make mixed gas easily by putting carbon dioxide gel into oxygen cylinder.
(6) "Carbon-dioxide snow (dry ice)" with carbon dioxide as a solid is used as a preservative of the corpses of a human being and the animal. And the temperature is about -80 degrees Celsius, when touch the carbon-dioxide snow directly, it may cause frostbite, then handling is difficult.
   Even more, when the carbon-dioxide snow sublimates and becomes the gas directly, the volume is swell out to 750 times, There is a danger that containers such as the PET bottle explode. Also, a quantity of the carbon dioxide generated by sublimating is much, the density of atmospheric carbon dioxide may reach 10-50%, it might cause carbon dioxide poisoning.
   The carbon dioxide gel of the present invention can use easily as a preservative anytime anywhere by applying to a corpse directly or bundling with a coffin,
(7) When keep industrial products in a sealed container or room, it seal after having supplied carbon dioxide gel enough, prevention of decay and an antirust effects are provided for cheapness and security.
(8) The carbon dioxide which is indispensable for the photosynthesis of the plant may be short in the greenhouse of a large quantity of plant husbandry or winter season, as a result, it occurs a phenomenon called "the carbon dioxide starvation" that the growth of the plant is confined.
   As the countermeasures, to supply carbon dioxide, it is conceivable to fertilize carbon dioxide, for example, burn propane gas and generates carbon dioxide and makes up for lack of the carbon dioxide, however, when it considers cost, danger, quantity of supplying, it is a very difficult problem. Thus, install carbon dioxide gel in a greenhouse or apply to a plant directly or spray, carbon dioxide is generated slowly and can be supplied, it can evade "carbon dioxide starvation".
   Even more particularly, cheap and safety is a big advantage when it uses the carbon dioxide gel.
(9) The carbon dioxide is indispensable for "photosynthesis" of the plant. Thus, while a plant does "photosynthesis", make an environment that carbon dioxide is produced around a plant continuously, by coating or atomizing the carbon dioxide gel to the branches and leaves of the plant, and promote photosynthesis and forces the plant.
As means to fertilize carbon dioxide, it is used well the water solution or solid of an alkali metal and the alkaline earth metal which absorb the carbon dioxide.
However, after having released carbon dioxide, there is an inconvenience that the compound of an alkali metal and the alkaline earth metal is left Then use the carbon dioxide gel, after having released dissolving carbon dioxide, the harmful ingredient is not left to the gel at all.

### [Helium gel]

(1) Helium gas can get into the microporosity, therefore it is used in nondestructive inspection system.
   When detect with a helium detector by injecting a helium gas into pipe conventionally, It can detect only some places in a short time. However, if use the helium gel of the present invention, the efficiency of the detection improves drastically, since the helium gas is generated over a wide area.
(2) As one of the party goods, helium gas is used for changing the quality of the voice (Donald Duck effect). Because speed of sound doubles approximately three in helium, this phenomenon is caused.
   Usually, put a mixture gas of 20% of oxygen 80% of helium in cylinder and use it, however if use the helium gel, the denaturation of the voice is easily enabled by applying or atomizing the gel to the hole of the nose.

### [Chlorine gel and Bromine gel]

The chlorine gel and bromine gel of the present invention can use as a strong oxidizer for long time.

Fig 5 is a figure explaining a process for preparing functional gel concerning the other embodiment, in this embodiment, set a gelling agent in container 6 beforehand, and supply a water (solution) in which micro-bubbles are produced through pipe 7 to the container 6.

### INDUSTRIAL APPLICABILITY

The functional gel of the present invention can be use as an antioxidant food, cosmetics, pharmaceutical products, various fields.
For example, it uses combustion promotion action when put a flammable gas or firedamp gas in gel.
The toxic gas derives the evasion action of the animals and plants and, it uses for the extermination of the animals and plants such as insect killing, sterilization or dry grass.
A noninflammability or the inert gas are used for fire extinguishing, fire prevention, flaming retardant operation.
Water-soluble gas and the non-water-soluble gas, it can select a gel material, coating agent and the materials of the excipient for maintaining the conditions that adopted in gel with bubble.
The case including an endotherm gas bubble is available as heat absorbency gel, and the case including a non-endotherm gas bubble is available as an insulation, thermal insulation, cold insulation, for example, wearing apparel, shoes, bedding, sporting goods, building materials, daily necessities, machines.
Nitrogen gas and the carbon dioxide gas are available for the growth of the plant.
All bubble gel are available in various fields depending on the gaseous kind to contain, such as pharmaceutical products, cosmetics, building materials, promotion restraint of the chemical reaction, catalytic action and control agent.

### DENOTATION OF REFERENCE NUMERALS

1...functional gel, 1a...gel, 1b...minute gas bubble, 2...airtight container, 3...high frequency source, 4...supersonic wave vibratory plate, 5...gas supply pipe, 6...gelating container, 7...flexible pipe, 8...tubular pump, P...case.

## Claims

1. A manufacturing method of functional gel **characterized** as, Put water or low viscosity solution into a container, feed hydrogen gas into the water or the low viscosity solution and generate minute hydrogen gas air bubbles, feed the water or the low viscosity solution in which minute hydrogen gas air bubbles are generated into an adjacent container using a tubular pump, add a gelling agent into the adjacent container while the micro-bubbles remain in the water or the solution, gelate the water or the solution and hold micro-bubbles of the hydrogen gas inside.

2. A manufacturing method of functional gel **characterized** as, Put water or low viscosity solution into a container, feed a gas (except hydrogen gas) into the water or the low viscosity solution and generate minute gas bubbles, feed the water or the low viscosity solution in which minute gas bubbles are generated into an adjacent container using a tubular pump, add a gelling agent into the adjacent container while the micro-bubbles remain in the water or the solution, gelate the water or the solution and hold micro-bubbles of the hydrogen gas inside.

3. A manufacturing method of functional gel **characterized** as, Put water or low viscosity solution into a container, feed a gas into the water or the low viscosity solution and generate minute gas bubbles, feed the water or the low viscosity solution in which minute gas bubbles are generated into an adjacent container using a tubular pump, a gelling agent is put in the adjacent container beforehand, mix the gelling agent in the container adjacent and the water or the low viscosity solution including minute air bubbles, gelate the water or the solution and hold micro-bubbles of the hydrogen gas inside.

4. A manufacturing method of functional gel according to claim 1∼3, in which an enteric coated material which is hard to dissolve in acid and easy to dissolve in alkali is dissolved in the water solution.
